# EUROPEAN PATENT SPECIFICATION

(11) **EP 1 478 448 B1**
(45) Date of publication and mention of the grant of the patent: **23.04.2008**
(21) Application number: 03706757.6
(22) Date of filing: 04.03.2003
(51) Int. Cl.: B01D 15/08

(54) **METHOD FOR MONITORING THE QUALITY OF A HERBAL MEDICINE**
VERFAHREN ZUR QUALITÄTSKONTROLLE EINER KRÄUTERMEDIZIN
METHODE DE CONTROLE DE LA QUALITE D'UNE PLANTE MEDICINALE

(30) Priority: 06.03.2002 GB 0205186
(43) Date of publication of application: 24.11.2004
(60) Divisional application: 06004780.0
(73) Proprietor: Summit Corporation Plc, Abingdon Oxfordshire OX14 4RY (GB)
(72) Inventor: NASH, Robert, James, Ystrad-Meurig, Ceredigion SY25 6AF (GB); PARRY, Hadyn, St Pierre, Farnham, Surrey GU9 8SR (GB); WATSON, Alison, Ann, Ystrad-Meurig, Ceredigion SY25 6AF (GB)
(74) Representative: Hutchins, Michael Richard
(86) International application number: PCT/GB2003/000906
(87) International publication number: WO 2003/074147

(56) References cited:
- EP-A- 0 886 143
- WO-A-99/34810
- ABIDI S L: "Chromatographic analysis of plant sterols in foods and vegetable oils" JOURNAL OF CHROMATOGRAPHY A, ELSEVIER SCIENCE, NL, vol. 935, no. 1-2, 23 November 2001 (2001-11-23), pages 173-201, XP004322070 ISSN: 0021-9673
- JANOS P: "Separation methods in the chemistry of humic substances" JOURNAL OF CHROMATOGRAPHY A, ELSEVIER SCIENCE, NL, vol. 983, no. 1-2, 3 January 2003 (2003-01-03), pages 1-18, XP004398944 ISSN: 0021-9673

## Description

### Field of the Invention

The invention relates to methods for monitoring the quality of a herbal medicine, and to processes for producing a herbal medicine.

### Background to the Invention

There is presently great interest in the use of herbal remedies and a growing acceptance from healthcare companies and the medical profession that the holistic approach of herbal medicinal products has value and can complement established therapy.

The revival of interest has been stimulated particularly by the successful use of standardized herbal medicinal products to treat chronic conditions for which conventional medicine is perceived to offer little therapeutic benefit. For example, standardized extracts of *Valeriana offcinalis* are used widely in Europe as sedatives, acting as natural alternatives to benzodiazepine drugs, while standardized extracts of *Ginkgo biloba* leaves are frequently prescribed in Germany and taken to alleviate cerebral ischemia. Other examples of herbal medicaments include Panax ginseng, *Allium sativum* (garlic), *Ginkgo biloba, Hypericum perforatum* (St John's wort), *Echinacea angustifolia* and *Aloe vera.*

A consequence of this tendency by the medical establishment to embrace the virtues of "herbal" products is that they will be subject to the same level of regulation as conventional drugs, to the benefit of the consumer. Thus, documented evidence of efficacy and safety, and of quality control for batch-to-batch reproducibility in levels of active components will be essential.

However, quality control of herbal medicines is difficult due to the complex nature and inherent non-uniformity of plant materials. The materials used in herbal and plant-based medicine are usually whole plants or parts or extracts thereof. Since plant and fungal materials contain many different chemical components the materials are complex mixtures. This makes it very difficult to standardize and control the quality of the materials. Moreover, many herbal medicines are mixtures of two or more plant-based components and are therefore mixtures of mixtures, so introducing a further level of complexity.

The active components of most herbal products remain under debate and inactive "markers" are often used for standardization. Such markers may be present in inactive products, or absent from active products.

Furthermore, the recipes and methods of manufacture used are often not uniform and may remain undisclosed. These factors make it very difficult to ensure that two samples of a given product, obtained from disparate sources and ostensibly identical, do in fact contain the same mixture of ingredients. This problem, which leads to difficulties in controlling the quality of such materials, has limited the use of certain herbal remedies even amongst herbal practitioners.

Other problems arise from the fact that the plants used in the practice of herbal medicine are frequently unavailable locally and therefore need to be obtained from sources which are remote from the end user. However, the supply of such plants from remote locations can be erratic and inaccurate, particularly because no detailed monographs including identity and quality standards exist for many such plants. The complex mixture of ingredients found in medicinal plants varies widely in type and concentration depending on many factors including the botanical source, the location where the plant is grown, the time of year when the plant is harvested, the conditions under which the material is stored and processed and the extraction procedure used.

There is therefore a need for sensitive processes which can profile herbal products and so establish a standard specification for a medicinal plant material which can be related to therapeutic activity, so permitting quality control in the production of herbal medicines and ideally quantifying the components known or likely to be active.

### Summary of the Invention

According to a first aspect of the present invention there is provided a method for monitoring the quality of a herbal medicine according to claim 1.

Preferably, the polar extract is fractionated prior to characterization. Any suitable method of fractionation may be employed, but in a preferred embodiment the polar extract is fractionated by: (a) ion-exchange chromatography to produce an extract enriched in ionic-compounds and a non-ionic residue; and then (b) chromatographic fractionation of the enriched extract of step (a) to yield one or more polar fractions comprising one or more ionic phytochemical(s). In such embodiments the chromatographic fractionation preferably comprises gas-liquid chromatography (GC), for example GC-MS. When GC is used, the enriched extract may be derivitized prior to chromatography.

In an optional variant of the method of the invention, the method further comprises the steps of: (i) scavenging the non-ionic residue for non-ionic species by subjecting the non-ionic residue to hydrophobic interaction or reversed-phase chromatography to produce a scavenged non-ionic extract depleted in sugars; and (ii) characterizing the scavenged extract.

The scavenged extract may be fractionated prior to characterization, for example by chromatographic fractionation to yield one or more scavenged fractions comprising one or more non-ionic phytochemical(s). Particularly preferred is high performance liquid chromatography (HPLC), for example HPLC-MS or HPLC-UVᵥᵢₛ.

The method may also optionally further comprise: (i) extracting a second sample of the herbal medicine or sequentially extracting the non-polar residue of the first sample with a non-polar solvent to produce a non-polar extract; and (ii) characterizing the non-polar extract.

The non-polar extract may be fractionated prior to characterization, for example by: (i) subjecting the non-polar extract to hydrophobic interaction or reversed-phase chromatography to produce an extract depleted in fats and chlorophyll; and (ii) chromatographically fractionating the depleted extract to yield one or more non-polar fractions comprising one or more non-polar phytochemical(s). The chromatographic fractionation may comprise high performance liquid chromatography (HPLC) and/or gas-liquid chromatography (GC), for example HPLC-MS/UVᵥᵢₛ and/or GC-MS

Any suitable form of characterization of the polar and/or non-polar extracts may be employed, including without limitation functional and/or physical and/or chemical characterization.

Where the extracts are functionally characterized, the characterization may comprises a biological assay, for example selected from *in vivo* or *in vitro* assays, enzyme inhibition assays (for example glycosidase and/or lipase inhibition), receptor binding assays, cellular assays (e.g. cell replication, cell-pathogen, cell-cell interaction and cell secretion assays), immunoassays, anti-microbial activity (e.g. bacterial and viral cell-binding and/or replication) assays, toxicity assays (e.g. LD₅₀ assays) or any combination thereof.

Where the extracts are physically characterized, the characterization may be selected from: (a) quantification of the phytochemical component(s); and/or (b) measurement of the purity of the constituents; and/or (c) determination of molecular weight (or molecular weight distribution or various statistical functions thereof in the case of fractions which comprise a plurality of different phytochemical constituents); and/or (d) determination of the molecular formula(e) (e.g. by nuclear magnetic resonance); and/or (e) spectral analysis.

Spectral analysis is particularly preferred, and may produce any or all of the following spectra:
(a) mass spectra (e.g. the mass to charge (m/z) value versus abundance), and/or
(b) chromatographic data (e.g. spectra, column retention times, elution profiles etc), and/or
(c) photodiode array (PDA) spectra (e.g. in both UV and visible ranges), and/or
(d) nuclear magnetic resonance (NMR) spectra (e.g. spectral data sets obtained *via* ¹H and/or ¹³C NMR).

When used according to the invention, the spectral analysis may be coupled with fractionation of the extract, for example by use of GC-MS and/or HPLC-PDA-MS.

Where the extracts are chemically characterized, the characterization may be selected from measurements of the chemical reactivity of phytochemical constituent(s), the solubility of phytochemical constituent(s), the stability and melting point of phytochemical constituent(s) or any combination thereof.

When employed according to the invention, the fractionation of the extract may be conducted under conditions which yield a defined fraction or an isolated (e.g. substantially pure) phytochemical.

In preferred embodiments of the invention the characterization yields a phytochemical profile, which may be analysed to: (a) determine whether one or more bioactive principal(s) are present in the sample(s); and/or (b) determine whether one or more bioactive marker(s) are present in the sample(s); and/or (c) determine whether it meets a standard specification.

The invention may find application in a method for identifying a bioactive principle in a herbal medicament. Preferably, the sample in this case is a blood sample which is obtained by administering a sample of the herbal medicine to a subject and then extracting a blood sample from the subject.

In another aspect, the invention provides a process for producing a herbal medicine comprising the step of monitoring the quality of the herbal medicine according to the methods of the invention as described above.

The quality control of herbal drugs comprising alkaloids is known for example from the Russian patent specification SU 1108354, however this document concerns only the determination of glaucine by addition of a marker, the papaverine, and does not concern the specific alkaloids of the present invention.

### Detailed Description of the Invention

### Definitions

Where used herein and unless specifically indicated otherwise, the following terms are intended to have the following meanings in addition to any broader (or narrower) meanings the terms might enjoy in the art:

The term *plant* is used herein in a broad sense to encompass not only plants *sensu stricto* but also fungi and bacteria.

The term *phytochemical* is used herein in a broad sense to encompass any chemical constituent of a plant, including macromolecules and small molecules. Important examples include alkaloids (for example pyrrolidines, piperidines, pyrrolizidine, indolizidines, tropanes and nortropanes), carbohydrate analogues, phenolic compounds, terpenoids, enzyme inhibitors, glycosides, nucleotides, amino acids, lipids and sugars. The phytochemicals may act *inter alia* as drugs, agrochemicals, templates for combinatorial chemistry, antioxidants, markers of botanical origin or quality, animal poisons, pesticides, cosmetics and food additives.

The term *herbal medicine* is used herein to define a pharmaceutical composition in which at least one active principle is not chemically synthesized and is a phytochemical constituent of a plant. In most cases, this non-synthetic active principle is not purified, but present together with other phytochemicals with which it is associated in the source plant. In some cases, however, the plant-derived *bioactive principle(s)* may be in a concentrated fraction or isolated (sometimes to high degrees of purity). In many cases, however, the herbal) medicine comprises a more or less crude extract, infusion or fraction of a plant or even an unprocessed whole plant (or part thereof), though in such cases the plant (or plant part) is usually at least dried and/or milled.

The term *bioactive principle* is used herein to define a phytochemical which is necessary or sufficient for the pharmaceutical efficacy of the herbal medicament in which it is comprised.

The term *characteristic* as used herein is intended to define data characterizing one or more aspects of a phytochemical constituent of a herbal medicine, such as its spectral properties, concentration, chemical structure or functional properties.

The term *phytochemical profile* is used herein to define a set of characteristics relating to different phytochemical constituents. Such a set of properties and/or features may also be referred to as a *chemical fingerprint.* The properties may include any or all of the properties discussed herein (see for example the section entitled "Fraction characterization"), and typically include spectral data such as mass spectra and/or PDA spectra.

The phytochemical profile may be derived from the analysis of a single plant species to detect the presence of a defined set of chemicals. Such profiling techniques may also be applied to plant extracts (and fractions thereof) in which case the profile may comprise spectral data relating to a collection of phytochemical constituents (typically greater than 5, preferably greater than 10, often greater than 20). Plant extracts and fractions which have been profiled in this way are referred to herein as *defined extracts* and *defined fractions,* respectively.

The term *bioactive marker* is used herein to define a characteristic (or a phytochemical profile) which is correlated with an acceptable degree of pharmaceutical activity.

The term *standard specification* is used herein to define a characteristic, or a phytochemical profiles, which is correlated with an acceptable quality of the herbal medicine. In this context, the term *quality* is used to define the overall fitness of the herbal medicament for its intended use, and may include for example the presence of one or more bioactive principles (at an appropriate concentration), the presence of one or more bioactive markers, a phytochemical profile which indicates the use of a particular source, condition, purity and an acceptable or unacceptable degree of contamination with undesirable supplements and/or contaminants.

The term *isolated* is used herein to indicate that the isolated material (e.g. the phytochemical) exists in a physical milieu distinct from that in which it occurs in nature. For example, the isolated material may be substantially isolated (for example purified) with respect to the complex cellular milieu in which it naturally occurs, particularly in the context of the libraries of the invention.

When *purified* material of the invention is specified herein the absolute level of purity is not critical and those skilled in the art can readily determine appropriate levels of purity according to the use to which the material is to be put. Preferred, however, are purity levels of 90% w/w, 99% w/w or higher.

In some circumstances, the isolated phytochemical forms part of a composition (for example a more or less crude extract containing many other substances) or buffer system, which may for example contain other components. In other circumstances, the isolated phytochemical may be purified to essential homogeneity, for example as determined spectrophotometrically, by NMR or by column chromatography (for example HPLC).

As used herein, the term *index of biological activity* is intended to define a characteristic or property which is correlated with a biological activity. For example, a particular constellation of reactive groups on a phytochemical may be used as a marker of toxicity, while the ability to interact with a particular receptor *in vitro* may be an index of pharmaceutical activity.

As used herein the terms *polar* and *non-polar* are applied as relative terms to solvents to indicate the degree to which they have an electric dipole moment and so display hydrophilicity (polar) or hydrophobicity (non-polar). They are used to extract polar and non-polar phytochemicals, respectively.

### Medicine samples

The medicine samples used in the methods of the present invention may be dried plant material, untreated aliquots of the herbal medicine in the form in which it is administered or offered for sale. Alternatively, the samples may be pre-processed in any of a wide variety of ways prior to characterization. Pre-processing may involve physical or chemical pre-processing, for example powdering, grinding, freezing, evaporation, filtration, pressing, spray drying, extrusion, supercritical solvent extraction and tincture production.

In cases where the herbal medicine is administered or sold in the form of a whole plant (or part thereof), the plant material may be dried prior to use. Any convenient form of drying may be used, including freeze-drying, spray drying or air-drying.

### Solvent extractions

Suitable polar solvents for use in the process of the invention include without limitation organic solvents such as organic alcohols. Preferred are ethanol and methanol, as well as ethanol/water or methanol/water mixtures.

Preferably, the polar solvent is selected from 51 to 80% ethanol/water, 31 to 50% ethanol/water, and up to 30% ethanol/water. Particularly preferred is a polar solvent which is approximately 50% ethanol/water.

Suitable non-polar solvents for use in the process of the invention include without limitation organic solvents such as hexane and dichloromethane (DCM) or chloroform. Particularly preferred is dichloromethane.

The conditions (time, temperature, degree of agitation etc.) under which the extraction(s) are performed can be readily determined empirically and vary according to the nature of the sample, the nature of any pre-processing and the solvent system selected.

### Chromatographic fractionation of the enriched extract

Chromatographic fractionation may comprise gas-liquid chromatography. Gas-liquid chromatography is a process whereby a complex mixture of volatile substances is separated into its constituents by partitioning the sample between an inert gas under pressure and a thin layer of non-volatile liquid coated on an inert support inside a heated column. In order to achieve a good separation of specific compounds in a mixture, it is crucial to use a column with the correct characteristics. The nature of the solid support, type and amount of liquid phase, method of packing, overall length and column temperature are important factors.

Those skilled in the art, by routine trial and error and by using common general knowledge, will be able readily to determine the appropriate column characteristics according to the circumstances, including *inter alia* the extract under study and the nature of the solvent used in the extraction. Particularly preferred, and useful in many circumstances, are capillary columns coated with a non-polar liquid phase (25m x 0.22mm id x 0.25µm BPX5 stationary phase, produced by SGE Ltd., or equivalents thereof).

Many compounds are unsuitable for direct injection into a gas chromatograph because of their high polarity, low volatility or thermal instability. Compounds that are highly hydroxylated are difficult to vapourise because of inter-molecular hydrogen bonding. However, by replacing the hydroxyl hydrogens with other chemical groups, they can be made sufficiently volatile for GC analysis.

The two most popular means of derivatising hydroxyl groups are acetylation and silylation, where acetylates [CH₃CO-O-R) or silyl ethers, e.g. trimethylsilyl (TMS) ethers [(CH₃)₃Si-O-R] are formed.

Thus, in embodiments where the enriched extract is chromatographically fractionated on an analytical scale the phytochemical constituents of the enriched extract are preferably derivitized, for example by acylation or silylation. Particularly preferred is trimethyl silyl (TMS) derivitization.

Chromatographic fractionation may also comprise ion exchange chromatography. Ion-exchange chromatography partially purifies ionic species to concentrate them and remove contaminating substances. Those skilled in the art, by routine trial and error and using common general knowledge, will be able readily to identify suitable column packing materials and mobile phase(s), which will depend *inter alia* on the quantities to be fractionated, the extracts under study and the nature of the solvent used in the extraction.

Particularly preferred in the methods of the present invention are strongly acidic cation exchange resins which can be used in either the free acid or hydrogen (H⁺) form or in the ammonium (NH₄⁺) salt form). These forms adsorb cations from solution and release an equivalent number of counter-ions back into solution (either H⁻ or NH₄⁺ ions, depending on the form used).

When used on a preparative scale, anion exchange chromatography and/or adsorption chromatography may also be used.

### Chromatographic fractionation of the scavenged extract

The optional scavenging process of the invention produces a scavenged non-ionic extract depleted in sugars which is chromatographically fractionated to yield one or more scavenged fractions comprising one or more non-ionic phytochemical(s).

The chromatographic fractionation preferably comprises high performance liquid chromatography (HPLC). With this technique, samples are dissolved in a suitable solvent and separated on a column using a solvent mixture that is pumped under pressure through the column. Those skilled in the art, by routine trial and error and using common general knowledge, will be able readily to identify suitable column packing materials, pumping pressures, flow rates and mobile phase(s) which will depend *inter alia* on the quantities to be fractionated, the plant material under study and the nature of the solvent used in the extraction.

Chromatographic fractionation on a preparative scale preferably comprises flash fractionation (e.g. normal phase flash fractionation) in conjunction with (e.g. followed by) high performance liquid chromatography (HPLC) (e.g. reverse phase HPLC). Flash fractionation is a form of preparative column chromatography which involves the application of pressure to speed solvent flow and can be carried out with a wide variety of supports.

The fractionation may also comprise gas-liquid chromatography (as described above in the section relating to fractionation of the enriched extract).

### Chromatographic fractionation of the depleted extract

The optional non-polar fractionation of the invention comprises subjecting a non-polar extract to hydrophobic interaction or reversed-phase chromatography to produce an extract depleted in fats and chlorophyll and chromatographically fractionating the depleted extract to yield one or more non-polar fractions comprising one or more non-polar phytochemical(s).

The analysis of the depleted extract preferably comprises high performance liquid chromatography (HPLC), although gas-liquid chromatography may also be used as an alternative or in conjunction with the HPLC.

### Physical state of the fractions

The physical state of the polar fraction (and optionally the scavenged and/or non-polar fractions) depends on the fractionation technique used in its preparation and will vary depending on the application.

In certain embodiments, an isolated phytochemical is essentially the sole phytochemical in any given isolated fraction. However, in some cases the isolated fractions contain a plurality of different phytochemicals, for example less than about 100, preferably less than about 15 but most preferably no more than about 5 different phytochemicals.

Particularly preferred are fractions containing isolated or purified phytochemicals, e.g. purified to about 90% purity (for example to over 90% or to over 99%, purity).

### Fraction characterization

The form the characterization takes depends on the nature of the medicine under study and the characterization techniques employed.

In general, any or all of the following approaches may be used:

### (a) Functional characterization

The functional characterization may comprise a biological assay. Biological assays may be carried out *in vivo* or *in vitro*, and may include enzyme inhibition assays (for example glycosidase and/or lipase inhibition). Other biological assays include receptor binding assays, cellular assays (including cell replication, cell-pathogen and cell-cell interaction and cell secretion assays), immunoassays, anti-microbial activity (e.g. bacterial and viral cell-binding and/or replication) assays and toxicity assays (e.g. LD₅₀ assays).

Functional characterization may also be carried out indirectly by a form of characterization which permits the identification of one or more indices of biological activity.

### (b) Physical characterization

This can take the form of quantification of the phytochemical component(s) present in any given fraction or at any other stage in the process, measurement of the purity of the constituents, determination of molecular weight (or molecular weight distribution or various statistical functions thereof in the case of fractions which comprise a plurality of different phytochemical constituents), determination of the molecular formula(e) (e.g. by nuclear magnetic resonance) and various spectral analyses.

Particularly useful spectral characteristics include:
- Mass spectra (e.g. the mass to charge (m/z) value versus abundance), and/or
- Chromatographic data (e.g. spectra, column retention times, elution profiles etc), and/or
- Photodiode array (PDA) spectra (e.g. in both UV and visible ranges), and/or
- Nuclear magnetic resonance (NMR) spectra (including spectral data sets obtained *via* ¹H and/or ¹³C NMR).

Spectral characterization can be coupled with the fractionation step. For example, GC-MS and HPLC-PDA-MS can be used (as described herein) to couple the fractionation with the obtention of mass spectral, UV-visible spectral and chromatographic spectral data.

Any or all of the above characteristics can be used to define a "chemical fingerprint" for any given sample (or any fraction or phytochemical constituent thereof).

### (c) Chemical characterization

This can take the form of measurements *inter alia* of the chemical reactivity of phytochemical constituent(s), their solubility, stability and melting point.

The invention will now be described with reference to specific examples. These are for illustrative purposes only, and are not intended to be limiting in any way to the scope of the monopoly claimed or the invention described.

### Exemplification

### Binding of ionic species

10g of dried plant material is put into a 250ml conical flask then enough 50% ethanol/water added to soak the plant material, allowing 2cm extra solvent on top. This is left for 15 hours or overnight to extract.

The extract is filtered using a Buchner funnel. The plant material is either discarded or kept for sequential extraction with dichloromethane (DCM). Preferably fresh material is used for the DCM step but if insufficient is available, a sequential extraction can be performed or might be used to further characterize the components).

Dowex 50 resin (50-100 mesh) is prepared by adding excess 2N HCI and soaking for a minimum of 15 minutes. The resin is then washed with excess deionized water to pH 7. The prepared resin is poured into 10x1cm columns and reservoirs attached. The columns are washed with 25ml of 50% aqueous ethanol to equilibrate the resin with the same solvent as used to prepare the plant samples.

For each column, the reservoir is filled with the extract which is allowed to pass slowly through the resin.

Fractions of approximately 30ml of the unbound sample are collected in a large vial, labelled and kept for HP-20 scavenging. The pH of the eluent is monitored which should be around 1 or 2. If it rises to 6 or 7 then the resin is exhausted. If this should happen, a little more resin is added to the top of the column and if necessary the whole sample is applied to the column again to ensure binding of all of the ionic components. After all of the sample has been applied to the column, it is washed with 75ml of 50% aqueous ethanol followed by 75ml of water. These washings are discarded. The water is used to remove the alcohol prior to eluting the bound constituents.

The column is eluted with 100ml of 2N ammonium hydroxide and this is collected in a 250ml round bottom flask. This is evaporated to 3-5ml on a rotary evaporator at less than 40°C and transferred to a weighed 7ml vial. The drying is completed by blowing down with nitrogen and/or freeze-drying. Care is taken to dry the samples on the same day and not to leave them sitting in the ammonia solution longer than necessary (typically less than 15 minutes) as compound degradation could otherwise occur. 1-3 mg of each dried sample is placed in GC vials and freeze dried again prior to analysis.

### Scavenging of Non-Ionic Species

This process utilises the unbound material from the Dowex 50 columns described above.

30ml of the unbound extract from a Dowex 50 column is collected in a large vial. A Sep-pak vacuum manifold is used with a Sep-pak cartridge (these contain HP-20 resin). The Sep-pak cartridges can be modified using a 5ml pipette tip to make a larger column.

A large vial or small beaker is placed under the cartridge to collect the waste. 5ml of the sample is loaded onto the Sep-pak cartridge. A gentle vacuum is applied to pull the sample through the cartridge at a steady drip. Once the sample has been loaded onto the HP-20 resin in the cartridge, the column is washed with 3ml of 25% methanol in water. This is collected in the same beaker/vial and the contents are then discarded. The purpose of this wash is to remove most of the sugars from the resin prior to elution. These are unwanted common metabolites that are generally present in large amounts in the aqueous ethanol plant extracts and if not removed these would interfere with the analysis of the samples.

The column is eluted with 5ml of 10% acetone in methanol and this sample is collected in a weighed 7ml vial. The sample is dried under vacuum and then freeze dried if necessary. The vial is reweighed and the sample made up to 10mg/ml in methanol. 150µl of the sample is transferred into a labelled HPLC and GC vials for analysis.

### Extraction of Non-Polar Components

A filter paper thimble is constructed and 10g of dried and ground plant material added or plant material dried after the removal of ionic chemicals. A few glass beads are placed in a 500ml round-bottom flask which is then placed in a heating mantle and 200ml of dichloromethane (DCM) added. The sample thimble is placed in a Soxhlet tube and this is attached to the round-bottom flask. 150ml of DCM is added to the sample in the Soxhlet tube. A condenser is placed on the top of the Soxhlet apparatus and the cooling water turned on. The heating mantle is switched on ensuring that a steady refluxing rate is established. At the end of the extraction the heating mantle is switched off. The system is allowed to cool for a further 30 minutes before turning off the water.

After allowing the extract to cool to ambient temperature, the Soxhlet apparatus is dismantled allowing any DCM remaining in the Soxhlet itself to siphon into the flask. The flask is removed from the mantle. 100ml of HP-20 resin is placed in a labelled 1000ml round-bottom flask and the DCM extract is then added. The HP-20 / extract is evaporated under vacuum on a rotary evaporator set at less than 40°C until dry. The dried resin is transferred to a 250ml conical flask and eluted with 3x100ml of 10% acetone in methanol. The solution is decanted through a filter into a pre-weighed 500ml round-bottom flask and rotary evaporated until dry. The round-bottom flask is re-weighed to determine the extract weight and the material is then made up to 10mg/ml in methanol and transferred to a labelled vial. The extract is filtered prior to analysis by HPLC-PDA/MS and GC-MS.

### Notes

### (a) HP-20 Resin

Diaion HP-20 (manufactured by Sumitomo Ltd) is a styrene-divinylbenzene polymer resin. It is hydrophobic and adsorbs lipophilic compounds and weak acids. The synthetic adsorbent HP and SP series are insoluble three-dimensional crosslinked polymers with macropores. They do not possess ion exchange or other functional groups, however they have a large surface area and are able to absorb a variety of organic substances by means of van der Waals' forces. The polymer matrix can be classified as either the aromatic (styrene-divinylbenzene) type or the acrylic (methacrylic) type.

Once compounds are adsorbed they can be washed off the resin by the application of a suitable solvent. HP-20 is used in the following manner to remove excessive amounts of fats and chlorophyll from dichloromethane (DCM) extracts of plants.

The solubilised extract is dried under vacuum onto the resin. The resin is eluted with methanol containing increasing amounts of acetone (up to 30% acetone). This is enough to wash off all compounds of interest whilst leaving fats and chlorophylls adsorbed onto the HP-20 resin. The HP-20 resin is cleaned for re-use by washing with acetone and hexane. This washes off all unwanted compounds and the resin can be used once again after a final wash with methanol.

For the scavenging of non-ionic components, the constituents of the extracts are more polar (water-soluble) than those in the dichloromethane extracts. Therefore, the HP-20 resin is used in a slightly different manner to separate sugars from the compounds of interest by washing these off the resin first using 25% methanol in water prior to the elution of the remaining bound material using 10% acetone in methanol. The key to the different uses of HP-20 resin lies in the polarity of the solvent systems used to elute the material adsorbed onto it.

### (b) Ion Exchange Chromatography

The ion exchange step allows concentration of ionic species to concentrate them and remove contaminating substances that could interfere with their analysis. Samples are initially processed by extraction using approximately 50% aqueous alcohol, which separates the polar constituents from the more non-polar components of each plant and denatures any proteins that may be present in the extract. The extracts are then processed by ion exchange chromatography which separates and concentrates the ionic compounds in each extract (predominantly alkaloids, amino acids and small amines) from the non-ionic compounds which would also be present in the extracts (mainly sugars, fats and most of the phenolic compounds). The samples are then analysed in enzyme assays, by GC-MS or HPLC.

The filtered extracts are loaded onto Dowex 50W-X8 resin, which is a polystyrene resin cross-linked with divinylbenzene. It is a strongly acidic cation exchanger which can be used in either the free acid or hydrogen (H⁺) form or in the salt form e.g. ammonium (NH₄⁺) salt. Both forms of the resin adsorb cations from solution and release an equivalent number of counter-ions back into solution (either H⁺ or NH₄⁺ ions, depending on the form of the resin used). In the H⁺ form, Dowex 50W-X8 resin adsorbs all ionic compounds from solution (except very strong acids), regardless of their charge, and this is the preferred form.

On adsorption of cations from the extract, protons are displaced from the resin causing the pH of the eluate to fall from pH 6.0 (the pH of the distilled water used to rinse the resin prior to use) to approximately pH 2.0, depending on the concentration of the sample. The more dilute the sample, the smaller the drop in pH. However, once the resin capacity has been reached, continued sample loading causes the pH to rise to that of the crude extract itself.

The Dowex 50W-X8 resin (50-100 mesh size) is prepared for use by washing with 2N HCI to ensure complete conversion to the H⁺ form. The excess acid is removed by extensive rinsing with distilled water. After the crude extract has been loaded onto the resin, the column is washed with distilled water to remove any unbound material until the pH of the eluate rises to that of the water itself The bound compounds are eluted with a 2N solution of ammonium hydroxide (NH₄⁺OH⁻). The column is washed to pH 6.0 with water and the ammonia is removed from the sample by evaporation under reduced pressure at 40°C using a rotary evaporator.

The material not bound by the ion exchange resin is reduced in volume by evaporation under reduced pressure for HP-20 scavenging of chemicals.

### (c) Gas Chromatography - Mass Spectrometry (GC-MS)

This technique is used to detect and quantify the constituents of the enriched, scavenged and depleted extracts.

Gas-liquid chromatography is a process whereby a complex mixture of volatile substances is separated into its constituents by partitioning the sample between an inert gas under pressure and a thin layer of non-volatile liquid coated on an inert support inside a heated column, In order to achieve a good separation of specific compounds in a mixture, it is crucial to use a column with the correct characteristics. The nature of the solid support, type and amount of liquid phase, method of packing, overall length and column temperature are important factors. Preferably capillary columns coated with a non-polar liquid phase (25m x 0.22mm id x 0.25µm BPX5 stationary phase, produced by SGE Ltd.) or equivalents thereof are used.

Many compounds are unsuitable for direct injection into a gas chromatograph because of either their high polarity, low volatility or thermal instability. Compounds that are highly hydroxylated are difficult to vapourise because of inter-molecular hydrogen bonding. However, by replacing the hydroxyl hydrogens with other chemical groups, they can be made sufficiently volatile for GC analysis. The two most popular means of derivatising hydroxyl groups are acetylation and silylation, where acetylates [CH₃CO-O-R] or silyl ethers, e.g. trimethylsilyl (TMS) ethers [(CH₃)₃Si-O-R] are formed. Preferred is the silylation of samples prior to analysis using Sigma Sil A (a mixture of trimethylchlorosilane, hexamethyldisilazane and pyridine 1:3:9) produced by the Sigma Chemical Company. Derivatisation is achieved by the addition of 100µl of Sigma Sil A to each mg of dried material in a sealed vial (the reagent degrades in the presence of water) and the reaction is completed by heating the samples at 60°C for 15 minutes.

The trimethylsilyl ethers in each derivatised sample are separated on the column using a temperature programme. A temperature programme is used as this allows the rapid separation of compounds of a very wide boiling range.

In electron impact mass spectrometry the effluent from the gas chromatograph, which contains the separated and vaporised compounds, is passed into the ion chamber of the mass spectrometer which is under a high vacuum. The molecules are bombarded by a beam of electrons accelerated from a filament which ionises and fragments them. Initially, one electron is removed from each molecule to form a positively charged molecular ion (M⁺, i.e. a radical cation). Breakage of bonds relative to bond strength occurs rapidly in the molecular ion to generate fragment ions. The manner in which molecules fragment is highly characteristic and can be used as a form of 'fingerprint' identification. The various ions are accelerated into the analyser portion of the mass spectrometer where they are sorted according to their mass to charge ratios (m/z values) which are equivalent to the molecular weights of the fragments. The ion signal is amplified by an electron multiplier and the mass spectrum is plotted from low to high mass. The m/z values are plotted against relative abundance of the ions to give the visual 'fingerprint'.

### (d) HPLC-PDA/MS/ELS (evaporative light scattering detection)

This technique is used to detect and quantify the constituents of the scavenged and depleted extracts. With this technique, samples are dissolved in a suitable solvent and separated on a column using a solvent mixture that is pumped under pressure through the column. Three detectors are used; a mass spectrometer, as described above, and a photodiode array system that measures whether the compounds absorb light at wavelengths in both the UV and visible ranges.

In the examples described above, a Waters Integrity^{™} HPLC-PDA/MS system fitted with a reverse phase C₈ HPLC column (50mm x 2.1 mm id x 3.5µm, Waters) was used. The rate of solvent flow through the column was 0.35ml/min and a linear gradient starting at 90% water and 10% acetonitrile (containing 0.01% trifluoroacetic acid) was used, rising to 100% acetonitrile over 6 minutes and held for a further 6.5 minutes.

Absorbance (photodiode array - PDA) data was collected from 200-600nm and mass spectral data collected between 71 and 600m/z.

## Claims

1. A method for monitoring the quality of a herbal medicine comprising the steps of:
(a) providing a first sample of the herbal medicine;
(b) extracting the sample with a polar solvent to produce: (i) an extract of polar phytochemicals selected from pyrrolidine, piperidine, pyrrolizidine, indolizidine, tropane and nortropane alkaloids; and (ii) a non-polar residue;
(c) characterizing the extract of polar phytochemicals.

2. The method of claim 1 wherein the extract of polar phytochemicals is fractionated prior to characterization by :
(a) ion-exchange chromatography to produce an extract enriched in ionic-compounds and a non-ionic residue; and then
(b) chromatographically fractionating, e.g. by gas-liquid chromatography, the enriched extract of step (a) to yield one or more polar fractions comprising one or more ionic phytochemical(s).

3. The method of claim 1 or claim 2 further comprising the steps of: (i) scavenging the non-ionic residue for non-ionic species by subjecting the non-ionic residue to hydrophobic interaction or reversed-phase chromatography to produce a scavenged non-ionic extract depleted in sugars; and (ii) characterizing the scavenged extract.

4. The method of any one of the preceding claims further comprising: (i) extracting a second sample of the herbal medicine or sequentially extracting the non-polar residue of the first sample with a non-polar solvent to produce a non-polar extract; and (ii) characterizing the non-polar extract.

5. The method of any one of the preceding claims wherein the polar and/or non-polar extracts are **characterized**:
(a) functionally; and/or
(b) physically; and/or
(c) chemically.

6. The method of claim 5 wherein: (a) the functional characterization comprises a biological assay selected from *in vivo* or *in vitro* assays; and/or enzyme inhibition assays glycosidase and/or lipase inhibition; and/or receptor binding assays; and/or cellular assays, e.g. cell replication, cell-pathogen, cell-cell interaction and cell secretion assays; and/or immunoassays; and/or anti-microbial activity, e.g. bacterial and viral cell-binding and/or replication assays; and/or toxicity assays, e.g. LD₅₀ assays, and/or (b) the physical characterization is selected from: quantification of the phytochemical component(s); and/or measurement of the purity of the constituents; and/or determination of molecular weight, or molecular weight distribution or various statistical functions thereof in the case of fractions which comprise a plurality of different phytochemical constituents; and/or determination of the molecular formula(e), e.g. by nuclear magnetic resonance; and/or spectral analysis, and/or (c) the chemical characterization comprises measurements of the chemical reactivity of phytochemical constituent(s); and/or the solubility of phytochemical constituent(s); and/or the stability and melting point of phytochemical constituent(s).

7. The method of any one of claims 2 to 6 wherein the fractionation of the extract of polar phytochemical yields a defined fraction or an isolated, e.g. substantially pure, polar phytochemical.

8. The method of any one of the preceding claims wherein the characterization yields a phytochemical profile, optionally further comprising the step of analysing the phytochemical profile to determine whether:
(a) one or more bioactive principle(s) or bioactive marker(s) are present in the sample(s); and/or
(b) it meets a standard specification.

9. A process for producing a herbal medicine comprising the step of monitoring the quality of the herbal medicine by a method as defined in any one of the claims 1 to 8.

## Patentansprüche

1. Methode zum Überwachen der Qualität eines Kräuterheilmittels umfassend die Schritte des:
(a) Bereitstellens einer ersten Probe des Kräuterheilmittels;
(b) Extrahierens der Probe mit einem polaren Lösungsmittel zum Herstellen: (i) eines Extrakts polarer Phytochemikalien ausgewählt unter Pyrrolidin-, Piperidin-, Pyrrolizidin-, Indolizidin-, Tropan- und Nortropanalkaloiden; und (ii) eines nichtpolaren Rückstands;
(c) Charakterisierens des Extrakts der polaren Phytochemikalien.

2. Methode nach Anspruch 1, wobei der Extrakt polarer Phytochemikalien vor dem Charakterisieren fraktioniert wird durch:
(a) Iononenaustauschchromatographie unter Bildung eines Extrakts, der an ionischen Verbindungen reich ist, und eines nichtionischen Rückstands; und daraufhin
(b) chromatographisches Fraktionieren, z.B. durch Gas-Flüssig-Chromatographie, des angereicherten Extrakts aus Schritt (a), um eine oder mehrere polare Fraktionen umfassend eine oder mehrere ionische Phytochemikalien zu ergeben.

3. Methode nach Anspruch 1 oder Anspruch 2, des Weiteren umfassend die Schritte des: (i) auffangendes Durchsuchen des nichtionischen Rückstands nach nichtionischen Spezies durch Unterwerfen des nichtionischen Rückstands einer hydrophoben Wechselwirkung oder Umkehrphasenchromatographie, um einen aufgefangenen nichtionischen Extrakt, der an Zuckern verarmt ist, zu erzeugen; und (ii) Charakterisieren des aufgefangenen Extrakts.

4. Methode nach einem der vorhergehenden Ansprüche, des Weiteren umfassend: (i) das Extrahieren einer zweiten Probe des Kräuterheilmittels oder das sequentielle Extrahieren des nichtpolaren Rückstands der ersten Probe mit einem nichtpolaren Lösungsmittel unter Bildung eines nichtpolaren Extrakts; und (ii) Charakterisieren des nichtpolaren Extrakts.

5. Methode nach einem der vorhergehenden Ansprüche, wobei der polare und/oder nichtpolare Extrakt:
(a) funktionell; und/oder
(b) physikalisch; und/oder
(c) chemisch
charakterisiert werden.

6. Methode nach Anspruch 5, wobei: (a) die funktionelle Charakterisierung einen biologischen Assay ausgewählt unter in vivo- oder in vitro-Assays; und/oder Enzymhemmungsassays, Glykosidase- und/oder Lipasehemmung; und/oder Rezeptorbindungsassays; und/oder Zellassays, z.B. Zellreplikations-, Zellkrankheitserreger-, Zell-Zell-Wechselwirkungs- und Zellabsonderungsassays; und/oder Immunassays; und/oder Assays bezüglich der antimikrobiellen Aktivität, z.B. Bakterien- und Viruszellbindungs- und/oder -replikationsassays; und/oder Toxizitätsassays z.B. LD₅₀-Assays umfasst und/oder (b) die physikalische Charakterisierung ausgewählt wird unter: der Quantifizierung der phytochemischen Komponente(n); und/oder dem Messen der Reinheit der Bestandteile; und/oder dem Bestimmen des Molekulargewichts oder der Molekulargewichtsverteilung oder verschiedener statistischer Funktionen derselben im Falle von Fraktionen, die mehrere verschiedene phytochemische Bestandteile umfassen; und/oder dem Bestimmen der molekularen Formel(n), z.B. durch Kernmagnetresonanz; und/oder Spektralanalyse und/oder (c) die chemische Charakterisierung Messbestimmungen der chemischen Reaktivität der phytochemischen Bestandteils bzw. der chemischen Bestandteile; und/oder der Löslichkeit des phytochemischen Bestandteils bzw. der phytochemischen Bestandteile; und/oder der Beständigkeit und des Schmelzpunkts des phytochemischen Bestandteils bzw. der phytochemischen Bestandteile umfasst.

7. Methode nach einem der Ansprüche 2 bis 6, wobei das Fraktionieren des Extrakts polarer Phytochemikalien eine definierte Fraktion oder eine isolierte, z.B. im Wesentlichen reine, polare Phytochemikalie ergibt.

8. Methode nach einem der vorhergehenden Ansprüche, wobei die Charakterisierung ein phytochemisches Profil ergibt, die wahlweise des Weiteren den Schritt des Analysierens der phytochemischen Profils umfasst, um zu bestimmen, ob:
(a) ein oder mehrere bioaktive Wirkstoff(e) oder bioaktive(r) Marker in der bzw. des Probe(n) vorliegen; und/oder
(b) es der Standardspezifikation entspricht.

9. Verfahren für die Herstellung eines Kräuterheilmittels umfassend den Schritt des Überwachens der Qualität des Kräuterheilmittels durch eine Methode, wie durch irgendeinen der Ansprüche 1 bis 8 definiert.

## Revendications

1. Méthode de contrôle de la qualité d'un médicament à base de plantes médicinales comprenant les étapes qui consistent à :
(a) fournir un premier échantillon du médicament à base de plantes médicinales ;
(b) extraire l'échantillon avec un solvant polaire en vue de produire : (i) un extrait de produits phytochimiques polaires sélectionnés parmi la pyrrolidine, la pipéridine, la pyrrolizidine, l'indolizidine, les alcaloïdes du tropane et du nortropane ; et (ii) un résidu non polaire ;
(c) caractériser l'extrait de produits phytochimiques polaires.

2. Méthode selon la revendication 1, dans laquelle l'extrait de produits phytochimiques polaires est, avant de procéder à la caractérisation, fractionné par :
(a) chromatographie sur échangeur d'ions pour produire un extrait enrichi en composés ioniques et un résidu non ionique ; et puis
(b) le fractionnement par voie chromatographique, par exemple par chromatographie gaz-liquide, de l'extrait enrichi obtenu à l'étape (a) pour donner une ou plusieurs fractions polaires comprenant un ou plusieurs produit(s) phytochimique(s) ionique(s).

3. Méthode selon la revendication 1 ou la revendication 2 comprenant, en outre, les étapes qui consistent à : (i) épurer le résidu non ionique pour les espèces non ioniques en soumettant le résidu non ionique à une interaction hydrophobe ou à la chromatographie en phase inverse en vue de produire un extrait non ionique épuré dépourvu de sucres ; et (ii) caractériser l'extrait épuré.

4. Méthode selon l'une quelconque des revendications précédentes comprenant, en outre : (i) l'extraction d'un second échantillon du médicament à base de plantes médicinales ou l'extraction séquentielle du résidu non polaire du premier échantillon avec un solvant non polaire en vue de produire un extrait non polaire ; et (ii) la caractérisation de l'extrait non polaire.

5. Méthode selon l'une quelconque des revendications précédentes, dans laquelle les extraits polaires et/ou non polaires sont **caractérisés** :
(a) fonctionnellement ; et/ou
(b) physiquement ; et/ou
(c) chimiquement.

6. Méthode selon la revendication 5, dans laquelle : (a) la caractérisation fonctionnelle comprend un dosage biologique sélectionné parmi des dosages *in vivo* ou *in vitro* ; et/ou des dosages d'inhibition d'enzymes ; d'inhibition de la glycosidase et/ou de la lipase ; et/ou des dosages de liaison aux récepteurs ; et/ou des dosages cellulaires, par ex. réplication cellulaire, cellule-agent pathogène, interaction cellule-cellule et dosages de sécrétion de cellules ; et/ou immmunodosages ; et/ou activité antimicrobienne, par ex. liaison aux cellules bactériennes et virales et/ou dosages de réplication ; et/ou dosages de toxicité, par ex. dosages DL₅₀, et/ou (b) la caractérisation physique est sélectionnée parmi : la quantification du(des) composant(s) phytochimique(s) ; et/ou la mesure de la pureté des constituants ; et/ou la détermination du poids moléculaire, ou la répartition du poids moléculaire ou diverses fonctions statistiques de ce dernier dans le cas des fractions qui comprennent une pluralité de constituants phytochimiques différents ; et/ou la détermination de la(des) formule(s) moléculaire(s) , par ex. par résonance magnétique nucléaire ; et/ou l'analyse spectrale, et/ou (c) la caractérisation chimique comprend les mesures de la réactivité chimique du(des) constituant(s) phytochimique(s) ; et/ou la solubilité du(des) constituant(s) phytochimique(s) ; et/ou la stabilité et le point de fusion du(des) constituant(s) phytochimique(s).

7. Méthode selon l'une quelconque des revendications 2 à 6, dans laquelle le fractionnement de l'extrait de produits phytochimiques polaires donne une fraction définie ou un produit phytochimique polaire isolé, par ex. substantiellement pur.

8. Méthode selon l'une quelconque des revendications précédentes, dans laquelle la caractérisation donne un profil phytochimique comprenant, optionnellement, en outre, l'étape qui consiste à analyser le profil phytochimique en vue de déterminer si :
(a) un ou plusieurs principe(s) bioactif(s) ou marqueur(s) bioactif(s) sont présents dans l'échantillon ou les échantillons ; et/ou
(b) s'il est conforme à une spécification standard.

9. Procédé de production d'un médicament à base de plantes médicinales comprenant l'étape qui consiste à contrôler la qualité dudit médicament à base de plantes médicinales par une méthode telle qu'elle est définie dans l'une quelconque des revendications 1 à 8.
